# EUROPEAN PATENT APPLICATION

(11) **EP 2 554 659 A1**
(43) Date of publication of application: **06.02.2013**
(21) Application number: 11765848.4
(22) Date of filing: 01.04.2011
(51) Int. Cl.: C12N 5/0735, C12N 5/07, C12N 5/10, A01K 67/027, C12N 15/12

(54) **METHOD FOR PREPARING ES CELLS**

(30) Priority: 02.04.2010 JP 2010086568
(71) Applicant: Riken, Wako-shi, Saitama 351-0198 (JP)
(72) Inventor: KOSEKI, Haruhiko, Yokohama-shi Kanagawa 230-0045 (JP); HASEGAWA, Takanori, Yokohama-shi Kanagawa 230-0045 (JP); ISHIKURA, Tomoyuki, Yokohama-shi Kanagawa 230-0045 (JP); MATSUDA, Masashi, Yokohama-shi Kanagawa 230-0045 (JP)
(74) Representative: Woods, Geoffrey Corlett
(86) International application number: PCT/JP2011/058448
(87) International publication number: WO 2011/125948

(57) **Abstract**

The present invention provides a method of producing a mammalian ES cell, including cultivating a mammalian inner cell mass in a medium containing adrenocorticotropic hormone, an FGF receptor inhibitor, an MEK activation inhibitor and a GSK3 inhibitor, and isolating a mammalian ES cell from the culture.

SEQUENCE LISTING

<110> RIKEN

<120> METHOD FOR PREPARING ES CELLS

<130> N.118158

<140> 11765848.4
<141> 2011-04-01

<150> JP 20100086568
<151> 2010-04-02

<160> 2

<170> PatentIn version 3.5

<210> 1
<211> 13
<212> DNA
<213> Homo sapiens

<400> 1
atgggggtgc ccg
13

<210> 2
<211> 30
<212> DNA
<213> Homo sapiens

<400> 2
gtgagtactc acaggctaag gacctgtgac
30

## Description

### Technical Field

The present invention relates to a production method of a mammalian ES cell, which is useful for the establishment of an ES cell of an NOD/SCID background mouse, a medium useful for the method, an NOD/SCID background mouse ES cell and the like.

### Background Art

ES cell (embryonic stem cell) was first established from a mouse blastocyst by Evans et al. in 1981. When chimeric mouse is generated by a method including injection of ES cells into a blastocyst and the like, ES cells differentiate into functional germ cells with high efficiency, and an offspring derived from the ES cell can be generated by crossing chimera mice. Such properties are most effectively utilized for the generation of knockout mouse, and it is well known that the analysis of gene function strikingly advanced due to the development of this technique.

At present, mouse ES cells are commercially available, and can be easily purchased. However, most of the commercially available ES cells are of 129 background. This is because the efficiency in the establishment of ES cells varies greatly among mouse strains. Generally, when compared to mouse of 129 background, the efficiency in the establishment of ES cells from other mouse is extremely low. Therefore, studies have been made to improve various culture conditions in an attempt to increase the efficiency of ES cell establishment.

Non-patent document 1 discloses that ACTH (adrenocorticotropic hormone) promotes clone expansion of mouse ES cells and maintains pluripotency of the ES cells.

Non-patent document 2 describes that the pluripotency of ES cell can be maintained by inhibiting FGF receptor, MEK and GSK3. It has been reported that ES cells could be established from NOD mouse under this condition (non-patent document 3).

NOD/SCID mouse is a well-known mouse generated by back crossing Scid mutant mouse to NOD background mouse (patent document 1). Since NOD/SCID mice cannot rearrange T cell receptor and immunoglobulin genes, they lack functional T cells and B cells in the peripheral blood and show low activity of NK cells, thus exhibiting severe complex immunodeficiency.

NOD/SCID mice exhibit severe immunodeficiency and do not reject various human tissues and cells that have been transplanted. Therefore, they are used as a basis for generating so-called "humanized mice". Particularly, NOD/SCID/common gamma deficient mice (NOG mice) obtainable by deleting common γ chain from NOD/SCID show various manners of immunodeficiency such as loss of T, B and NK cells, disappearance of the activity of complement, deficiency in the function of macrophages and dendritic cells (patent document 2). Therefore, NOG mice have strikingly high engraftment potential of human cells and tissues as compared to conventional mice, and permit differentiation of transplanted human stem cells into mature cells. Thus, they are useful for the generation of various humanized mouse models (non-patent document 4).

### [Document List]

### [patent documents]

patent document 1: JP-A-Hei-9-94040
patent document 2: JP-B-3753321

### [non-patent documents]

non-patent document 1: Genes to Cells, vol.9, pp.471-477, 2004
non-patent document 2: Nature, vol.453, pp.519-523, 2008
non-patent document 3: Biology of Reproduction, vol.81, no.6, pp.1147-1153, 2009
non-patent document 4: Blood, vol.100, no.9, pp.3175-3182, 2002

### SUMMARY OF THE INVENTION

### Problems to be Solved by the Invention

Conventionally, a human environment is reconstructed in a humanized mouse by generating transgenic mice having introduced human gene, mating the mice for several generations to finally obtain an NOG mouse expressing the human gene. To achieve this, a long time of 2 years or more and a large amount of labor are generally required.

The present inventors have considered that this problem can be solved by establishing an ES cell from an NOD/SCID background mouse and introducing human gene thereinto to generate the human gene-introduced NOD/SCID background mouse. To establish the ES cell from the NOD/SCID background mouse, therefore, an inner cell mass (ICM) of NOD/SCID mouse was cultured in a medium containing LIF according to the method of establishing an ES cell from a 129 background mice, but an ES cell could not be established. Therefore, the present inventors tried addition of ACTH, reported in non-patent document 1 to increase ES cell establishment efficiency, to the medium, but could not establish ES cell of NOD/SCID mouse. Furthermore, an FGF receptor inhibitor, an MEK activation inhibitor and a GSK3 inhibitor were added to the medium according to the method that established an ES cell from an NOD mouse (non-patent document 4). As a result, an ES cell could be established, but the obtained cell showed high differentiation tendency, and did not proliferate while maintaining pluripotency.

Thus, the present inventors have found a new problem that an ES cell capable of proliferation while maintaining pluripotency is difficult to establish from an NOD/SCID background mouse by conventionally-known methods.

It is therefore an object of the present invention to provide a production method of a mammalian ES cell, which can establish an ES cell capable of proliferation while maintaining pluripotency, even from an NOD/SCID background mouse.

### Means of Solving the Problems

The present inventors have conducted intensive studies in an attempt to solve the aforementioned problems. As a result, they have found that an ES cell of an NOD/SCID background mouse capable of proliferation while maintaining pluripotency can be established by cultivating an inner cell mass of the NOD/SCID background mouse in the presence of ACTH, an FGF receptor inhibitor, an MEK activation inhibitor and a GSK3 inhibitor, which resulted in the completion of the present invention.

Accordingly, the present invention relates to the following.
[1] A method of producing a mammalian ES cell, comprising cultivating a mammalian inner cell mass in a medium containing adrenocorticotropic hormone, an FGF receptor inhibitor, an MEK activation inhibitor and a GSK3 inhibitor, and isolating a mammalian ES cell from the culture.
[2] The production method of [1], wherein the mammal is a mouse.
[3] The production method of [2], wherein the mouse is an NOD/SCID background mouse.
[4] The production method of [1], wherein the medium further comprises LIF.
[5] The production method of [1], wherein the FGF receptor inhibitor is SU5402.
[6] The production method of [1], wherein the MEK activation inhibitor is PD184352.
[7] The production method of [1], wherein the GSK3 inhibitor is CHIR99021.
[8] An isolated NOD/SCID background mouse ES cell capable of proliferation while maintaining pluripotency.
[9] A medium containing adrenocorticotropic hormone, an FGF receptor inhibitor, an MEK activation inhibitor and a GSK3 inhibitor.
[10] The medium of [9], further comprising LIF.

### Effect of the Invention

Using the method of the present invention, a mammalian ES cell capable of proliferation while maintaining pluripotency can be established efficiently and, particularly, an ES cell of an NOD/SCID background mouse capable of proliferation while maintaining pluripotency can be established.
Using the ES cell of the NOD/SCID background mouse, the ES cell can be variously engineered genetically in vitro, and a genetically-modified NOD/SCID background mouse can be produced with ease. Therefore, the ES cell of the NOD/SCID background mouse is useful as a basis for the production of a humanized mouse.

### Brief Description of the Drawings

Fig. 1 shows a chimeric mouse obtained by microinjection of ES cells of NOD/SCID mouse into a C57BL/6 derived blastocyst embryo.
Fig. 2 shows a chimeric mouse obtained by microinjection of ES cells of NOD/SCID mouse into a C57BL/6 derived blastocyst embryo.
Fig. 3 shows offspring mice derived from ES cells of NOD/SCID mouse obtained by mating a germ line chimeric mouse, obtained by microinjection of ES cells of NOD/SCID mouse into a C57BL/6 derived blastocyst embryo, with a wild-type mouse.
Fig. 4 shows offspring mice derived from ES cells of NOD/SCID mouse obtained by mating a germ line chimeric mouse, obtained by microinjection of ES cells of NOD/SCID mouse into a C57BL/6 derived blastocyst embryo, with a wild-type mouse.
Fig. 5 shows a chimeric mouse obtained by microinjection of ES cells of NOD/SCID/common gamma KO mouse into a C57BL/6 derived blastocyst embryo.
Fig. 6 shows a chimeric mouse obtained by microinjection of ES cells of NOD/SCID/common gamma KO mouse into a C57BL/6 derived blastocyst embryo.
Fig. 7 shows a colony form at 2 days from replating BALB/cA mouse derived ES cells (clone No. 7) after maintenance culture in comparison medium 2 (containing an FGF receptor inhibitor, an MEK activation inhibitor and a GSK3 inhibitor, and free of ACTH).
Fig. 8 shows a colony form at 2 days from replating BALB/cA mouse derived ES cells (clone No. 6) after maintenance culture in comparison medium 2 (containing an FGF receptor inhibitor, an MEK activation inhibitor and a GSK3 inhibitor, and free of ACTH).
Fig. 9 shows a colony form at 2 days from replating BALB/cA mouse derived ES cells (clone No. 2) after maintenance culture in CCM+3i medium (containing ACTH, an FGF receptor inhibitor, an MEK activation inhibitor and a GSK3 inhibitor).
Fig. 10 shows a colony form at 2 days from replating BALB/cA mouse derived ES cells (clone No. 1) after maintenance culture in CCM+3i medium (containing ACTH, an FGF receptor inhibitor, an MEK activation inhibitor and a GSK3 inhibitor).
Fig. 11 shows a schematic diagram of a human erythropoietin gene knock-in vector.
Fig. 12 shows a chimeric mouse obtained by microinjection of ES cells of NOD/SCID mouse into a C57BL/6 derived blastocyst embryo having knocked-in human erythropoietin gene.

### Description of Embodiments

The present invention provides a method of producing a mammalian ES cell, comprising cultivating a mammalian inner cell mass in a medium containing adrenocorticotropic hormone, an FGF receptor inhibitor, an MEK activation inhibitor and a GSK3 inhibitor, and isolating a mammalian ES cell from the culture.

The method of the present invention is characterized by the use of a medium containing adrenocorticotropic hormone, an FGF receptor inhibitor, an MEK activation inhibitor and a GSK3 inhibitor, for cultivation of a mammalian inner cell mass and, as for other culture conditions, the present invention can be performed according to a conventionally known method for establishing ES cells from an inner cell mass of a mammal. Refer to, for example, Method in Molecular Biology, volume 329, "Embryonic Stem Cell Protocol", second edition, Humana Press and the like, for the conventionally-known production method of a mammalian ES cell.

ES cell (embryonic stem cells) refers to a stem cell cell line produced from an inner cell mass of a part of the embryo in the blastocyst stage, which is an early developmental stage of an animal. The ES cell produced by the method of the present invention can proliferate in vitro while maintaining pluripotency. Pluripotency means an ability to differentiate into any cell or a progenitor cell thereof constituting the living organism including germ line.

Examples of the mammal include, but are not limited to, experiment animals such as rodents (e.g., mouse, rat, hamster, guinea pig and the like), rabbit and the like, domestic animals such as swine, bovine, goat, horse, sheep, mink and the like, companion animals such as dog, cat and the like, primates such as human, monkey, Macaca fascicularis, Macaca mulatta, marmoset, orangutan, chimpanzee and the like. A preferable mammal is mouse.

Mouse strain is not particularly limited and includes, for example, C57BL/6, DBA2, B6C3F₁, BDF₁, B6D2F₁, BALB/c, ICR, 129 and the like. In addition, the present invention can also be used for the production of ES cells of mutant mice such as NOD mouse, SCID mouse, NOD/SCID mouse and the like. NOD mouse is a known diabetes model mouse inbred from outbred Jcl-ICR mouse. SCID mouse is a known immunodeficient mouse showing severe combined immunodeficiencies. NOD/SCID mouse is a mouse obtainable by backcrossing SCID mouse to NOD mouse. For example, SCID mouse is mated with NOD mouse according to a methodology known to those of ordinary skill in the art, such as backcrossing according to Cross Intercross method (Inbred Strains in Biomedical Research, M.F.W. Festing, 1979, ISBN 0-333-23809-5, The Macmillan Press, London and Basingstoke), F1 mice thereof are mated again to give F2 mouse. The serum immunoglobulin level is measured and a mouse with undetectable immunoglobulin level is selected. The mouse is mated again with NOD mouse. This operation is repeated at least 9 times (Cross Intercross method) to give the NOD/SCID mouse. The production method of NOD/SCID mouse is disclosed in JP-A-Hei-9-94040. NOD mouse, SCID mouse and NOD/SCID mouse are all commercially available from CLEA Japan, Inc.

In the present specification, the NOD/SCID mouse and a mouse obtainable by further introduction of one or more mutations into NOD/SCID mouse is referred to as "NOD/SCID background mouse". As the mouse obtained by further introduction of one or more mutations into NOD/SCID mouse, NOD/SCID/common gamma deficient mouse (NOG mouse) and the like can be mentioned. The production method of NOG mouse is disclosed in, for example, JP-B-3753321. NOG mouse is commercially available from Central Institute for Experimental Animals.

An inner cell mass can be obtained by a method known per se. For example, male and female mammals are naturally mated, and the embryo is recovered 2 - 3 days later by washing the uterus and oviduct with a medium. To increase egg collection efficiency, superovulation is preferably induced in advance in the female by a hormone treatment. The collected embryo is developed up to the blastocyst stage by cultivation in an appropriate medium (e.g., M16). The culture temperature in this case is generally about 30 - 40°C, preferably about 37°C. The CO₂ concentration is generally about 1 - 10%, preferably about 7%. The humidity is, for example, generally about 70 - 100%, preferably about 95 - 100%. The culture period to reach the blastocyst stage is generally 1 - 3 days. An inner cell mass is contained in the blastocyst.

The obtained inner cell mass is transferred into a medium containing adrenocorticotropic hormone, an FGF receptor inhibitor, an MEK activation inhibitor and a GSK3 inhibitor and cultivated. As long as the cultivated cell contains an inner cell mass, the inner cell mass may or may not be isolated. That is, an isolated inner cell mass may be cultured, or a blastocyst containing an inner cell mass may be directly cultured. The method of isolating an inner cell mass from the blastocyst is well known to those of ordinary skill in the art. For example, blastocyst is treated with acidic Tyrode to dissolve zona pellucida, and then subjected to an immunosurgery using antiserum and complement, after which trophectoderm cells are removed to isolate an inner cell mass from the blastocyst.

As the basal medium of the medium used for the method of the present invention, a medium generally used for the culture of mammalian ES cells can be used, which is not particularly limited as long as a mammalian ES cell can be produced by the method of the present invention. For example, GMEM, DMEM, EMEM, RPMI-1640, α-MEM, F-12, F-10, M-199, HAM, ATCC-CRCM30, DM-160, DM-201, BME, SFM-101, Fischer, McCoy's 5A and the like can be mentioned. Alternatively, a medium modified for ES cell culture and the like can also be used, or a mixture of the above-mentioned basal media can also be used.

The adrenocorticotropic hormone (ACTH) to be contained in the medium is a known peptide hormone. ACTH use in the present invention is mammalian ACTH. Examples of the mammal include experiment animals such as rodents (e.g., mouse, rat, hamster, guinea pig and the like), rabbit and the like, domestic animals such as swine, bovine, goat, horse, sheep, mink and the like, companion animals such as dog, cat and the like, primates such as human, monkey, Macaca fascicularis, Macaca mulatta, marmoset, orangutan, chimpanzee and the like, but are not particularly limited thereto as long as a mammalian ES cell can be produced by the method of the present invention. Furthermore, a peptide having a physiological activity substantially equivalent to that of ACTH, namely, a peptide having an amino acid sequence constituting the above-mentioned mammalian ACTH, except that one or more amino acids are deleted, substituted and/or added, and having a physiological activity equivalent to that of the corresponding full length peptide can also be used, and such peptides are also encompassed in ACTH. For example, it is known that ACTH is a peptide consisting of 39 amino acids (ACTH(1-39)), wherein N-terminal 1 - 24 amino acids are common to various animals, 25 - 33 amino acids vary depending on the species, and N-terminal 1 - 18 shows an adrenocorticotropic action.- In the present invention, ACTH(1-39) as well as fragments thereof [ACTH(1-24), ACTH(11-24) and the like] can also be used. The above-mentioned ACTH and fragments thereof are described in documents (e.g., US-B-4,415,546; Kazutomo Imahori et al.: Seikagaku-Jiten (3rd edition) pages 1178 - 1179 (1998) Tokyo-Kagaku-Doujin), or commercially available as reagents, or can be produced according to the method described in a document. For example, such peptides can be produced by a peptide synthesis method for the construction of a desired amino acid sequence, which is well known to those of ordinary skill in the art.

In the method of the present invention, the concentration of ACTH contained in the medium is not particularly limited as long as a mammalian ES cell can be produced by the method of the present invention. It is generally 1 nM - 100 µM, preferably 1 - 30 µM.

The FGF receptor inhibitor to be contained in the medium typically refers to a small molecular weight compound or polypeptide that inhibits mammalian (e.g., mouse, human) FGFR1 and/or FGFR2. Therefore, an FGF receptor inhibitor can be an inhibitor of one member, some members, or all members of the FGF receptor family of a mammal (e.g., mouse, human). Examples of the FGF receptor include, but are not limited to, FGFR1, FGFR2, FGFR3 and FGFR4. Many FGF receptor inhibitors are known, and examples thereof include, but are not limited to, SU5402 (3-[3-(2-carboxyethyl)-4-methylpyrrole-2-methylidenyl]-2-indolinone) (see, for example, Bernard-Pierrot (2004) Oncogene 23: 9201-9211), PD173074 (1-t-butyl-3-(6-(3,5-dimethoxyphenyl)-2-(4-diethylaminobutylamino)-pyrido[2,3-d]pyrimidin-7-yl)urea) (see, for example, Moffa et al. (2004) Mol. Cancer Res. 2:643-652) and the like. In addition, soluble mammalian (e.g., mouse, human) FGF receptors (FGFR1, FGFR2 etc.) and specific neutralization antibodies against mammalian (e.g., mouse, human) FGF receptors (FGFR1, FGFR2 etc.) are also useful as FGF receptor inhibitors. The FGF receptor inhibitor is preferably SU5402.

In the method of the present invention, the concentration of an FGF receptor inhibitor to be contained in the medium is not particularly limited as long as a mammalian ES cell can be produced by the method of the present invention, and can be appropriately determined by those of ordinary skill in the art depending on the kind of the FGF receptor inhibitor. For example, when SU5402 is used as an FGF receptor inhibitor, the concentration thereof is generally 0.1 - 20 µM, preferably 0.5 - 10 µM, particularly 1 - 5 µM. When PD173074 is used as an FGF receptor inhibitor, the concentration thereof is generally 1 - 200 nM, preferably 5 - 100 nM, particularly 10 - 50 nM.

The MEK activation inhibitor to be contained in the medium refers to general MEK activation inhibitors. Therefore, an MEK activation inhibitor refers to any inhibitor of the activation of the mammalian (e.g., mouse, human) MEK family member protein kinases including MEK1, MEK2 and MEK3, for example, MEK1 activation inhibitor, MEK2 activation inhibitor and MEK3 activation inhibitor. The MEK activation inhibitor can inhibit activation of one member, some members, or all members of the MEK kinase family. Examples of appropriate MEK activation inhibitors include, but are not limited to, MEK1 activation inhibitors PD184352 (2-(2-chloro-4-indo-phenylamino)-N-cyclopropylmethoxy-3,4-difluorobenzamido) and PD98059 (2'-amino-3'-methoxyflavone), MEK1 and MEK2 activation inhibitors U0126 (1.4-diamino-2,3-dicyano-1,4-bis[2-amino-phenylthio]butadiene) and SL327 (α-[amino[(4-aminophenyl)thio]methylene]-2-(trifluoromethyl)benzeneacetonitrile), as well as those described in Davies et al. (2000) (Davies SP, Reddy H, Caivano M, Cohen P. Specificity and mechanism of action of some commonly used protein kinase inhibitors. Biochem J. 351, 95-105), which are already known in the pertinent technical field. Particularly, PD184352 was found to show high specificity and high effectiveness when compared to other known MEK activation inhibitors. Other MEK activation inhibitors and the class of MEK activation inhibitors are described in Zhang et al. (2000) Bioorganic & Medicinal Chemistry Letters; 10:2825-2828. A preferable MEK activation inhibitor is PD184352.

In the method of the present invention, the concentration of an MEK activation inhibitor to be contained in the medium is not particularly limited as long as a mammalian ES cell can be produced by the method of the present invention, and can be appropriately determined by those of ordinary skill in the art depending on the kind of the MEK activation inhibitor. For example, when PD184352 is used as an MEK activation inhibitor, the concentration thereof is generally 0.05 - 10 µM, preferably 0.25 - 5 µM, particularly 0.5 - 2.5 µM.

The GSK3 inhibitor to be contained in the medium refers to an inhibitor of one or more GSK3 enzymes. Therefore, a GSK3 inhibitor can inhibit one member, some members, or all members of the GSK3 enzyme family of mammals (e.g., mouse, human). The GSK3 enzyme family is well known, and examples thereof include, but are not limited to, GSK3-α and GSK3-β. Many mutants are described (see, for example, Schaffer et al.; Gene 2003; 302(1-2): 73-81). In a particular embodiment, GSK3-β is inhibited. GSK3-α inhibitor is also appropriate, and the GSK3 inhibitor generally used in the present invention inhibits the both. A wide range of GSK3 inhibitors are known. Examples of the GSK3 inhibitor include, but are not limited to, CHIR99021 (6-{2-[4-(2,4-dichloro-phenyl)-5-(4-methyl-1H-imidazol-2-yl)-pyrimidin-2-ylamino]-ethylamino}-nicotinonitrile), CHIR98014 (2-[[2-[(5-nitro-6-aminopyridin-2-yl)amino]ethyl]amino]-4-(2,4-dichlorophenyl)-5-(1H-imidazol-1-yl)pyrimidine), AR-A0144-18, TDZD-8 (4-benzyl-2-methyl-1,2,4-thiadiazolysine-3,5-dione), SB216763 (3-(2,4-dichlorophenyl)-4-(1-methyl-1H-indol-3-yl)-1H-pyrrole-2,5-dione) and SB415286 (3-[(3-chloro-4-hydroxyphenyl)amino]-4-(2-nitrophenyl)-1H-pyrrole-2,5-dione) and the like. Other inhibitors are known, and are useful for the present invention. Examples of the GSK3 inhibitor are described in Bennett C et al., J. Biol. Chem., vol. 277, No. 34, August 23, 2002, pages 30998-31004 and Ring DB et al., Diabetes, vol. 52, March 2003, pages 588-595. Furthermore, the structure of the active site of GSK3-β has been characterized, and important residues that interact with specific or nonspecific inhibitors have been identified (Bertrand et al.; J Mol Biol. 2003; 333 (2):393-407). By this structural property, a further GSK inhibitor can be identified easily. A preferable GSK3 inhibitor is CHIR99021.

In the method of the present invention, the concentration of a GSK3 inhibitor to be contained in the medium is not particularly limited as long as a mammalian ES cell can be produced by the method of the present invention, and can be appropriately determined by those of ordinary skill in the art depending on the kind of the GSK3 activation inhibitor. For example, when CHIR99021 is used as a GSK3 inhibitor, the concentration thereof is generally 0.01 - 100 µM, preferably 0.1 - 20 µM, more preferably 0.3 - 10 µM.

The medium to be used in the method of the present invention may further contain LIF. Since LIF has an activity to suppress differentiation of, for example, mouse ES cell, use of LIF in the production or culture of mouse ES cell is well known. However, it is known that LIF is not necessary when producing ES cells depending on the kind of mammal (e.g., human), and therefore, LIF is not essential. Thus, whether the medium contains LIF can be appropriately determined by those of ordinary skill in the art in reference to the known conditions for establishing ES cells and based on the difference in the animal species and the like. The LIF usable in the present invention is mammalian LIF. Examples of the LIF include LIF of human (JP-A-Hei-1-502985), mouse (JP-A-Hei-1-502985), sheep (JP-A-Hei-4-502554), swine (JP-A-Hei-4-502554), bovine (JP-A-Hei-8-154681) and the like.

When LIF is used in the method of the present invention, its concentration in the medium is not particularly limited as long as an ES cell can be produced by the method of the present invention. It is generally 10 - 10⁶ units/ml, for example, 10² - 10⁵ units/ml, preferably 5×10² - 5×10⁴ units/ml.

In addition, the medium to be used in the method of the present invention may further contain serum. While the kind of the serum is not particularly limited as long as a mammalian ES cell can be produced by the method of the present invention, it is preferably derived from the above-mentioned mammal (for example, fetal bovine serum etc.). The serum is preferably decomplementated. An alternative serum additive (for example, Knockout Serum Replacement (KSR) (manufactured by Invitrogen) etc.) may also be used. While the concentration of the serum is not particularly limited as long as a mammalian ES cell can be produced by the method of the present invention, it is generally 0.1 - 30 (v/v)%. In the method of the present invention, serum is not an essential factor. Establishment of an ES cell using a serum-free medium has been reported (Method in Molecular Biology, volume 329, "Embryonic Stem Cell Protocol", second edition, page 91-98, 2006, Humana Press; Genesis. 2004 Jun;39(2):100-4), and those of ordinary skill in the art can produce a mammalian ES cell by the method of the present invention by reference to the culture conditions disclosed therein and using a serum-free medium.

The medium to be used in the method of the present invention can contain additives known per se to be used for culture of mammalian cells. While the additive is not particularly limited as long as a mammalian ES cell can be produced by the method of the present invention, it is, for example, growth factors (for example, insulin etc.), an iron source (for example, transferrin etc.), polyamines (for example, putrescine etc.), minerals (for example, sodium selenate etc.), saccharides (for example, glucose etc.), organic acids (for example, pyruvic acid, lactic acid etc.), serum proteins (for example, albumin etc.), amino acids (for example, L-glutamine etc.), reducing agents (for example, 2-mercaptoethanol etc.), vitamins (for example, ascorbic acid, d-biotin etc.), antibiotics (for example, streptomycin, penicillin, gentamicin etc.), buffering agents (for example, HEPES etc.) and the like. Said additive is preferably contained in a concentration range known per se.

In the method of the present invention, an inner cell mass may be cultured on feeder cells. While the kind of the feeder cell is not particularly limited as long as a mammalian ES cell can be produced by the method of the present invention, a feeder cell known per se, which is used for cultivating an ES cell while maintaining pluripotency, can be used and, for example, fibroblast (mouse embryonic fibroblast, mouse fibroblast cell line STO etc.) can be mentioned. The feeder cell is preferably inactivated by a method known per se, for example, radiation (gamma ray etc.), a treatment with an anticancer agent (mitomycin C etc.) and the like. In the method of the present invention, a feeder cell is not an essential factor. Establishment of an ES cell under feeder-free conditions has been reported (Method in Molecular Biology, volume 329, "Embryonic Stem Cell Protocol", second edition, page 91-98, 2006, Humana Press), and those of ordinary skill in the art can produce a mammalian ES cell by the method of the present invention by reference to the culture conditions disclosed therein and under feeder-free conditions.

As the cell culture conditions in the method of the present invention, culture conditions generally used for producing a mammalian ES cell can be used. For example, the culture temperature is generally about 30 - 40°C, preferably about 37°C. The CO₂ concentration is generally about 1 - 10%, preferably about 7%. The humidity is generally about 70 - 100%, preferably about 95 - 100%. The culture period of an inner cell mass is a period sufficient for an ES cell to be produced from the inner cell mass (generally 1 week or longer), during which the culture can be continued while appropriately repeating the passage.

The operation after the start of the culture is explained in more detail, for example, as described below.

It is preferable to not change the medium after the start of the culture of a mammalian inner cell mass in a medium containing adrenocorticotropic hormone, an FGF receptor inhibitor, an MEK activation inhibitor and a GSK3 inhibitor until the growth of the inner cell mass occurs. The period until the occurrence of the growth of the inner cell mass is generally about 4 - 7 days from the start of the culture.

When the inner cell mass has grown to a diameter of about 100 µm, cell aggregate is dissected into about 3 - 4 aggregates with an injection needle and the like and the culture is continued. Culture is further continued while exchanging the medium with a fresh medium every day until the cell aggregates start to grow again. The period until the start of the growth of the aggregates again is generally about 4 - 5 days.

The well confirmed to show growth of the cell aggregates is treated with trypsin-EDTA to disperse the cell aggregates, and the detached cells are seeded in a new plate. In this way, passage is repeated by a method well known in the technical field except that the cells are cultivated in a medium containing adrenocorticotropic hormone, an FGF receptor inhibitor, an MEK activation inhibitor and a GSK3 inhibitor.

After repeating passages in this manner, a cell colony appears. A colony of undifferentiated ES cells shows a packed, characteristic form, which is clearly different from the colony form of the differentiated cells. Those of ordinary skill in the art can clearly and visually recognize the colony of undifferentiated ES cells based on the form of colony. Accordingly, using such colony form as a clue, for example, undifferentiated ES cells can be isolated from the culture by selectively picking up the colony of undifferentiated ES cells under a microscope with Pasteur pipette, micromanipulator and the like. The "culture" refers to a resultant product obtained by cultivating the cells, which includes cell, medium, and in some cases, cell secretory component and the like.

Whether the ES cell obtained by the method of the present invention maintains pluripotency can be confirmed by a method known per se. For example, a part of the obtained cells is introduced into a host embryo, and the birth or no birth of a chimeric animal is analyzed, whereby whether the cell maintains pluripotency can be confirmed. When the ES cells obtainable by the method of the present invention are introduced into a host embryo, they can contribute to the normal development of a chimeric animal. Moreover, whether the obtained cell also maintains differentiation potency into a germ line can be confirmed by mating the obtained chimeric animal with a wild-type animal, confirming whether the offspring animals include an animal having a genotype transmitted from the ES cell. The ES cells obtainable by the method of the present invention also maintain differentiation potency into a germ line.

Alternatively, whether the obtained ES cells maintain pluripotency can be confirmed by analyzing the expression of a marker gene characteristic of undifferentiated ES cell such as Nanog, Oct4 and Sox-2 and the like. Moreover, whether the obtained cell also maintains pluripotency can be confirmed by analyzing the ability to generate teratoma including all 3 germ layers (i.e., endoderm, mesoderm and ectoderm).

By such confirmation tests, a mammalian ES cell capable of proliferation while maintaining pluripotency can be certainly obtained.

The ES cell that can be produced by the method of the present invention is capable of proliferating 10-fold or more in 3 days.

Using the method of the present invention, a mammalian ES cell capable of proliferation while maintaining pluripotency can be efficiently established. Particularly, an ES cell of an NOD/SCID background mouse capable of proliferation while maintaining pluripotency can be established. The present invention also provides an isolated ES cell of an NOD/SCID background mouse capable of proliferation while maintaining pluripotency. It was not possible to establish an ES cell of an NOD/SCID background mouse by conventional production methods of ES cells. However, an ES cell of an NOD/SCID background mouse was successfully established for the first time by the method of the present invention.

In the present specification, "isolated" means that an operation to remove cells other than the object cell has been performed. The purity of cell X in the "isolated cell X" is generally not less than 70%, preferably not less than 80%, more preferably 90%, most preferably 100%, of the total number of cells.

An ES cell of an NOD/SCID background mouse capable of proliferation while maintaining pluripotency is provided, for example, as a culture in a medium used for the aforementioned method of the present invention.

Since the ES cell obtainable by the method of the present invention can proliferate for a long term while maintaining pluripotency, a genetically modified ES cell, for example, ES cell transfected with a particular exogenous gene, ES cell with deficiency of a particular gene and the like, can be produced by modifying the gene of said ES cell by a method known per se. Examples of the method of gene transfer into the ES cell obtainable by the method of the present invention include a method comprising introducing a vector constructed to permit functional expression of a particular gene into the ES cell. Examples of the method of obtaining a pluripotent stem cell with deficiency of a particular gene include homologous recombination using a targeting vector (gene targeting method).

The ES cells obtainable by the method of the present invention have the capability of differentiating into all somatic cells constituting a living organism including germ lines; conventionally-known all experimental techniques and methods applicable to ES cells can be applied to the ES cells; using the ES cells, it is possible to produce various functional cells, tissues, mammals and the like. Provided that ES cells genetically modified by the above-described method are used, it is possible to produce various genetically modified functional cells, tissues, mammals and the like.

Production of a mammal using the ES cell that can be obtained by the method of the present invention can be performed in accordance with, for example, a method known per se such as a method using a chimeric embryo.

For example, first, an ES cell which can be obtained by the method of the present invention is introduced into a host embryo to obtain a chimeric embryo. The animal species of the "host" is preferably the same as the animal species of the ES cell introduced. Examples of the "embryo" include, but are not limited to, blastocysts, 8-cell stage embryos and the like.

An "embryo" can be obtained by mating a female animal that received a superovulation treatment with a hormone preparation (for example, PMSG, which has FSH-like action, and hCG, which has LH action, are used) and the like with a male animal and the like. As methods of introducing an ES cell into a host embryo, the microinjection method, aggregation method and the like are known, and any method can be used.

Next, the chimeric embryo is transferred to the uterus or oviduct of the host animal to obtain a chimeric animal (excluding humans). The host animal is preferably a pseudo-pregnant animal. A pseudo-pregnant animal can be obtained by mating a female animal in the normal sexual cycle with a male animal emasculated by vasoligation and the like. The host animal having the transferred chimeric embryo will become pregnant and bear a chimeric animal (excluding humans).

Furthermore, it is possible to obtain an animal (excluding humans) harboring the gene derived from the ES cells (an animal derived from the ES cells) by mating the chimeric animal (excluding humans) with a normal animal or within the chimeric animals, and selecting an individual harboring the gene derived from the ES cells from among the individuals of next generation (F1). In selecting an animal (excluding humans) harboring a gene derived from ES cells, various characters can be used as indicators; for example, body color and coat color are used as the indicators. It is also possible to perform the selection by extracting DNA from a portion of the body and performing Southern blot analysis or PCR assay.

By using the above-described method, for example, it is possible to obtain an animal (transgenic animal) harboring a particular exogenous gene from ES cells transfected with the exogenous gene. Also, from ES cells with deficiency of a particular gene, it is possible to obtain a gene-deficient heterozygotous animal. Furthermore, by propagating the gene-deficient heterozygotous animals obtained, it is possible to obtain a gene-deficient homozygotous animal.

Particularly, using the ES cell of an NOD/SCID background mouse that can be produced by the method of the present invention, a transgenic NOD/SCID background mouse and a gene-deficient NOD/SCID background mouse can be produced easily by performing various gene manipulations of the ES cell in vitro. Thus, the cell is useful as a basis for the production of a humanized mouse.

The present invention also provides a medium containing adrenocorticotropic hormone, an FGF receptor inhibitor, an MEK activation inhibitor and a GSK3 inhibitor. The definition and content of adrenocorticotropic hormone, an FGF receptor inhibitor, an MEK activation inhibitor and a GSK3 inhibitor to be contained in the medium of the present invention, the kind of the basal medium for the medium, and the kind and content of the factor that may be further contained are as described in the explanation of the aforementioned method of the present invention. The medium of the present invention is useful for practicing the above-mentioned method of the present invention.

The contents disclosed in any publication cited in the present specification, including patents and patent applications, are hereby incorporated in their entireties by reference, to the extent that they have been disclosed herein.

The present invention is explained in more detail in the following by referring to Examples, which are not to be construed as limitative.

### Examples

### [Example 1]

### (method)

NOD/SCID mice were naturally mated. On day 2, embryos were collected from the oviduct. Eight-cell embryos were isolated, and cultured in M16 medium in a CO₂ incubator (7% CO₂, 37°C) for 2 days to allow development to the blastocyst stage. The blastocyst stage embryos were transferred to a 4-well dish with a bed of mitomycin C-treated mouse embryonic fibroblasts [MEFs], and cultured in a medium (CCM+3i medium) having the following composition. The medium was not exchanged until the embryos attached to the MEFs and the inner cell mass (ICM) started to grow (about 1 week).

### <CCM+3i medium>

Glasgow minimal essential medium (GMEM, Sigma)
10% (v/v) FCS;
100 µM 2-mercaptoethanol (Nacalai tesuque);
1× non-essential amino acids (Invitrogen);
1 mM sodium pyruvate (Invitrogen);
2000 units/mL LIF (ESGRO, Invitrogen);
10 µM ACTH; and
3i (FGF receptor inhibitor SU5402, 2 µM; MEK activation inhibitor PD184352, 0.8 µM; and GSK3 inhibitor CHIR99021, 3 µM)

When the inner cell mass (ICM) grew to a diameter of about 100 µm, the cell aggregate was dissected into 3 or 4 parts using a 27 gauge injection needle, and the culture was continued. The medium was changed every day while waiting for the regrowth of the cell aggregates (about 4 - 5 days). The cells in a well showing confirmed cell aggregate growth were treated with trypsin (0.05%)-EDTA, transferred to a 24-well dish with a bed of MEFs, and continuously cultured in CCM+3i medium. Since the ES cell-like growth was observed, the cells were re-seeded in a 24-well plate or 3.5 cm dish depending on the cell amount, and continuously cultured in CCM+3i medium.

### (results)

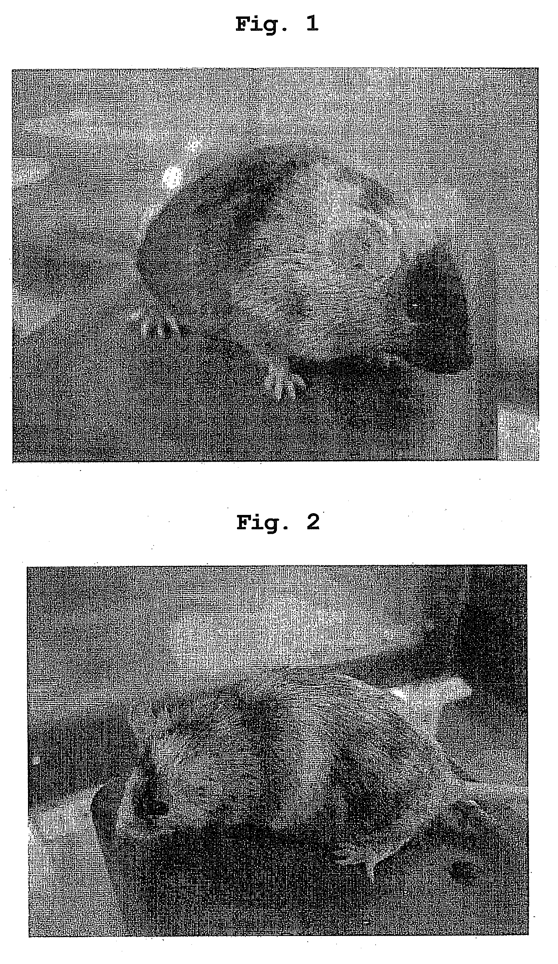

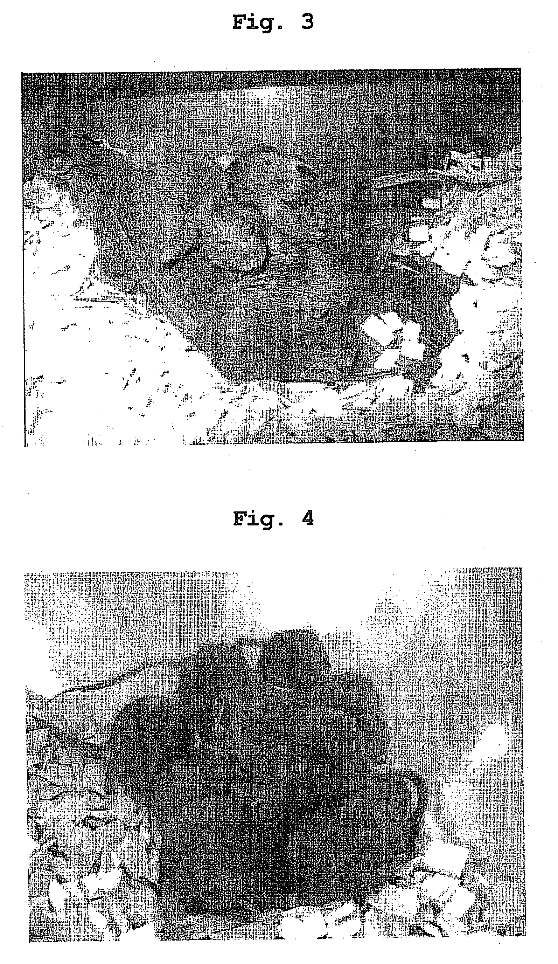

28 preimplantation embryos were cultured to give 9 ES-like cells (5 female clones, 4 male clones). Of these, 2 male-derived clones were microinjected into C57BL/6 derived blastocyst embryos 40 times or 42 times, respectively, to give chimeric mice [6 mice (female 3, male 3) and 9 mice (female 3, male 6), respectively] (Figs. 1, 2). Of these, male mice were checked for transmission to the germ line, and transmission was confirmed in 2 mice (Figs. 3, 4).

### [Comparative Example 1]

Using a medium having the following composition (comparison medium 1) instead of the CCM+3i medium, NOD/SCID/common gamma KO mouse preimplantation embryos (21 embryos) were cultured using the same protocol as in Example 1. As a result, 2 clones of ES-like cells were successfully produced.

### <comparison medium 1>

Glasgow minimal essential medium (GMEM, Sigma)
10%(v/v) FCS;
100 µM 2-mercaptoethanol (Nacalai tesuque);
1× non-essential amino acids (Invitrogen);
1 mM sodium pyruvate (Invitrogen);
10000 units/mL LIF (ESGRO, Invitrogen); and
10 µM ACTH
(Comparison medium 1 is different from CCM+3i medium since it does not contain 3i, and the concentration of LIF is 10000 units/mL.)

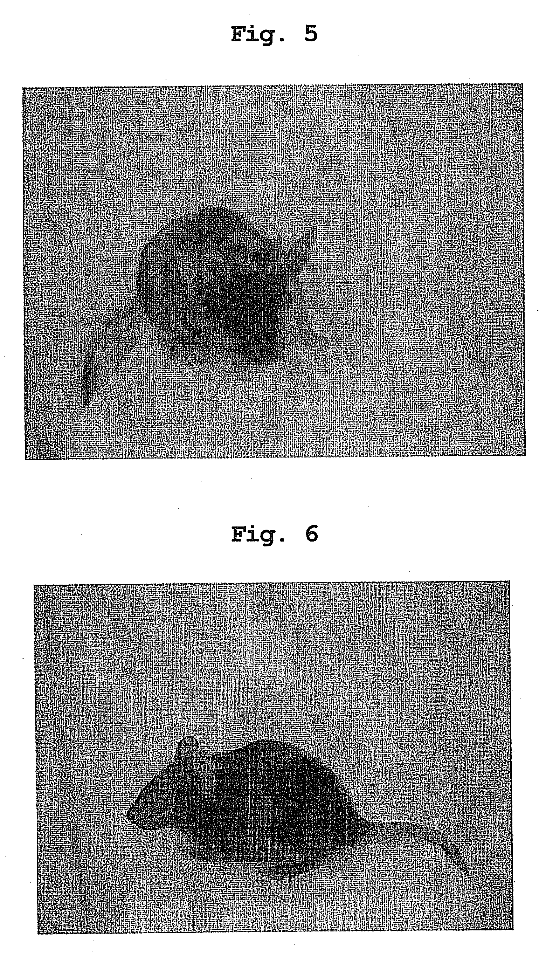

However, one of the clones showed a strong differentiation tendency, and the ES-like morphology could not be maintained unless cloning was continued. Microinjection was repeated 100 times or more to obtain only two chimeric mice (Figs. 5, 6); however, transmission to the germ line did not occur. As for the other clone, cell proliferation ceased on the way and the clone disappeared.

### [Comparative Example 2]

According to the same protocol as in Example 1, ES cells were established from a preimplantation embryo of BALB/cA mouse. For comparison, establishment of ES cell was tried using a medium having the following composition (comparison medium 2) instead of the CCM+3i medium.

### <comparison medium 2>

Glasgow minimal essential medium (GMEM, Sigma)
10%(v/v) FCS;
100 µM 2-mercaptoethanol (Nacalai tesuque);
1× non-essential amino acids (Invitrogen);
1 mM sodium pyruvate (Invitrogen);
2000 units/mL LIF (ESGRO, Invitrogen); and
3i (FGF receptor inhibitor SU5402, 2 µM; MEK activation inhibitor PD184352, 0.8 µM; and GSK3 inhibitor CHIR99021, 3 µM)
(Comparison medium 2 is different from CCM+3i medium since it does not contain ACTH.)

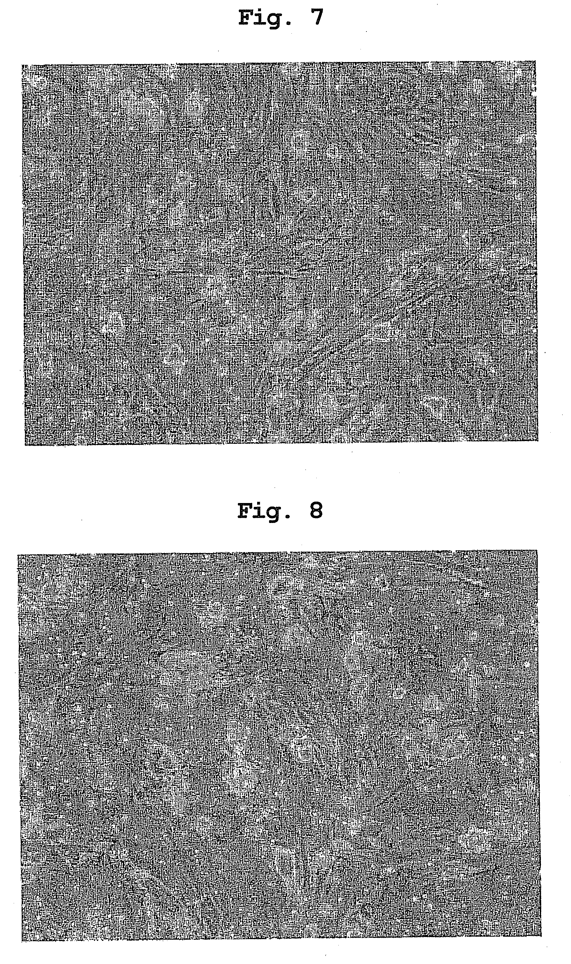

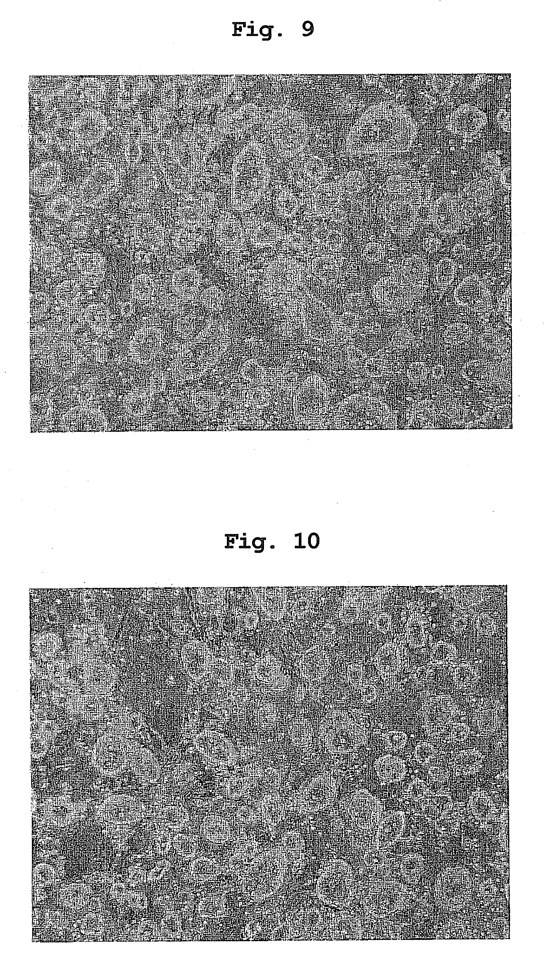

Using comparison medium 2, 3 ES-like cells were obtained from 8 preimplantation embryos, and using CCM+3i medium, 5 ES-like cells were obtained from 12 preimplantation embryos. However, the cells obtained by using comparison medium 2 all showed extremely low proliferative capacity, and were unsuitable for substantial use (Figs. 7, 8, 9, 10). Therefore, comparison medium 2 was also considered unsuitable for establishing ES cells for a strain other than 129, C57BL/6.

### [Example 2]

### (method)

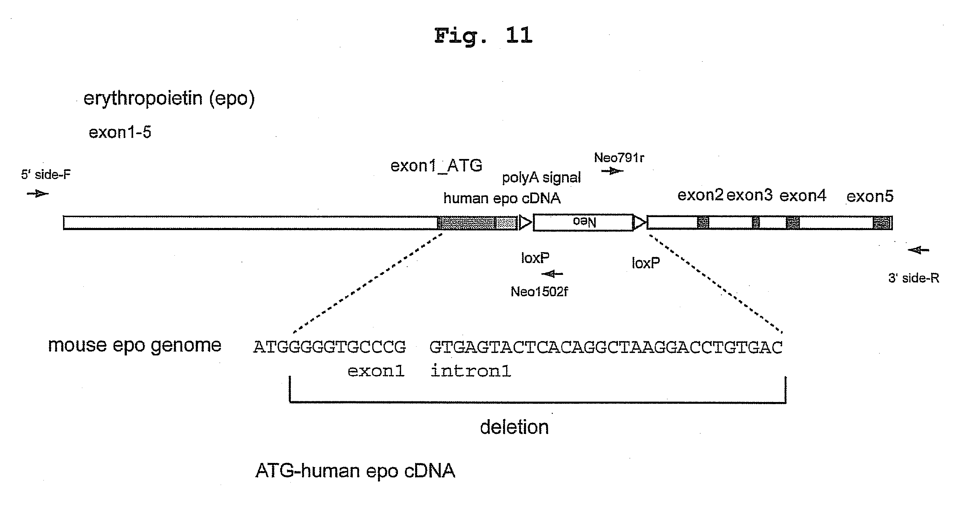

NOD/SCID derived ES cells were expanded on MEFs (feeder cells) to 2X10⁷ cells in the following medium (CCM+3i medium), and a linearized human erythropoietin gene knock-in vector (Fig. 11) was introduced into the cells according to general protocol using an electroporation method.

### <CCM+3i medium>

Glasgow minimal essential medium (GMEM, Sigma)
10% (v/v) FCS;
100 µM 2-mercaptoethanol (Nacalai tesuque);
1× non-essential amino acids (Invitrogen);
1 mM sodium pyruvate (Invitrogen);
2000 units/mL LIF (ESGRO, Invitrogen);
10 µM ACTH; and
3i (FGF receptor inhibitor SU5402, 2 µM; MEK activation inhibitor PD184352, 0.8 µM; and GSK3 inhibitor CHIR99021, 3 µM)

The cells after gene transfer were cultured again on MEFs in CCM+3i medium on six 6-cm culture plates, the medium was changed to a selection medium (CCM+3i medium added with 150 µg/ml G418) 48 hr later, and the culture was continued for 4 - 5 days. Thereafter, G418 resistant colonies were picked up under a microscope, and the obtained cell aggregates were treated with trypsin (0.05%)-EDTA, transferred to a 48-well plate with a bed of MEFs, and continuously cultured in the above-mentioned selection medium.

The genotype was determined using half the cells grown in each well, and the remaining half was cryopreserved. Using the primer (Neo791r/3'side-R) shown in Fig. 11, homologous recombinants were screened for. As a result, the possibility was shown that 16 clones out of 24 clones may be homologous recombinants. The genotype thereof was verified using 5'-side F/Neo1502r primer at that time to show all were homologous recombinants.

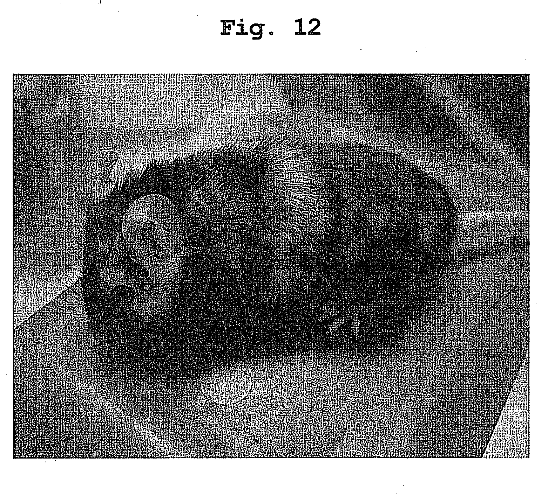

Of those recombinants, 3 clones were each subjected to 40 times of microinjection into B57BL6 derived blastocyst embryo, and 6 (♂3♀3), 9 (♂6♀3) and 6 (♂3♀3) chimeric mice were obtained (Fig. 12).

### Industrial Applicability

Using the method of the present invention, an ES cell of an NOD/SCID background mouse capable of proliferation while maintaining pluripotency and transmission capacity to a germ line can be established.
Using an ES cell of an NOD/SCID background mouse, the ES cell can be variously engineered genetically in vitro, and a genetically-modified NOD/SCID background mouse can be produced with ease. Therefore, an ES cell of an NOD/SCID background mouse is useful as a basis for the production of a humanized mouse.

This application is based on a patent application No. 2010-086568 filed in Japan (filing date: April 2, 2010), the contents of which are incorporated in full herein.

## Claims

1. A method of producing a mammalian ES cell, comprising cultivating a mammalian inner cell mass in a medium containing adrenocorticotropic hormone, an FGF receptor inhibitor, an MEK activation inhibitor and a GSK3 inhibitor, and isolating a mammalian ES cell from the culture.

2. The production method according to claim 1, wherein the mammal is a mouse.

3. The production method according to claim 2, wherein the mouse is an NOD/SCID background mouse.

4. The production method according to claim 1, wherein the medium further comprises LIF.

5. The production method according to claim 1, wherein the FGF receptor inhibitor is SU5402.

6. The production method according to claim 1, wherein the MEK activation inhibitor is PD184352.

7. The production method according to claim 1, wherein the GSK3 inhibitor is CHIR99021.

8. An isolated NOD/SCID background mouse ES cell capable of proliferation while maintaining pluripotency.

9. A medium containing adrenocorticotropic hormone; an FGF receptor inhibitor, an MEK activation inhibitor and a GSK3 inhibitor.

10. The medium according to claim 9, further comprising LIF.
